(19) Europäisches Patentamt / European Patent Office / Office européen des brevets

(11) **EP 2 976 639 B1**

(12) **EUROPEAN PATENT SPECIFICATION**

(45) Date of publication and mention
of the grant of the patent:
**31.10.2018 Bulletin 2018/44**

(21) Application number: **13712205.7**

(22) Date of filing: **21.03.2013**

(51) Int Cl.:
***G01N 33/497*** (2006.01)

(86) International application number:
**PCT/EP2013/055883**

(87) International publication number:
**WO 2014/146714 (25.09.2014 Gazette 2014/39)**

(54) **METHOD AND APPARATUS FOR DETECTING BREATH ALCOHOL CONCENTRATION BASED ON ACOUSTIC BREATH SAMPLER**

VERFAHREN UND VORRICHTUNG ZUR ERKENNUNG EINER ATEMALKOHOLKONZENTRATION AUF BASIS EINER AKUSTISCHEN ATEMPROBENNAHME

PROCÉDÉ ET APPAREIL POUR LA DÉTECTION DE LA CONCENTRATION D'ALCOOL DANS L'HALEINE FAISANT APPEL À UN ÉCHANTILLONNEUR ACOUSTIQUE D'HALEINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.01.2016 Bulletin 2016/04**

(73) Proprietor: **2045 Tech S.r.l.**
**I-30021 Caorle (VE) (IT)**

(72) Inventors:
• **Barbetta, Marco**
**35028 Piove di Sacco (PD) (IT)**
• **Penzo, Fabio**
**30021 Caorle (VE) (IT)**

(74) Representative: **Spadaro, Marco et al**
**Cantaluppi & Partners S.r.l.**
**Via Strobel, 8**
**20133 Milano (IT)**

(56) References cited:
**WO-A1-2012/087187    GB-A- 2 018 596**
**US-A- 4 314 564    US-A1- 2003 176 803**
**US-A1- 2013 021 153**

• **MENZ B: "Vortex flowmeter with enhanced accuracy and reliability by means of sensor fusion and self-validation", MEASUREMENT, INSTITUTE OF MEASUREMENT AND CONTROL. LONDON, GB, vol. 22, no. 3-4, 12 November 1997 (1997-11-12), pages 123-128, XP004123097, ISSN: 0263-2241, DOI: 10.1016/S0263-2241(97)00075-4**

**Description**

TECHNICAL FIELD

The present invention relates to the field of detectors of gas concentration in breath, comprising both devices and methods. In particular, the present invention relates to portable and/or fixed breath alcohol concentration detectors. Such devices allow estimating the blood alcohol concentration indirectly by measuring the gaseous alcohol concentration in breath. Some of the devices belonging to this category are named Breathalyzer™, which is a trademark of Draëger Safety, A.G.

BACKGROUND OF THE INVENTION

The detection of blood alcohol concentration is useful to estimate a person's sobriety and to determine if such person is able to drive. Many methods for measuring blood alcohol concentration are based on an indirect measurement. The measurement is performed on breath, and the gaseous ethanol concentration in breath (BrAC) is measured and then the blood alcohol concentration (BAC) is recovered by means of a well-known relation.

Methods for measuring gas ethanol concentration can be based on infrared spectroscopy, semiconductor sensors or fuel-cell sensors. All of them sample a part of the exhaled gas through a mouthpiece.

The first method brings accurate results, but is neither scalable to a portable device nor cost-effective for a consumer application.

The second method is less expensive, but quite inaccurate and needs electric power to be delivered to heating elements for the sensors, which makes this method less effective for ultra-low power portable devices.

An example of implementation of this second method is described in EP1046910. The apparatus uses a semiconductor ethanol sensor, which the gas blown by the user is delivered to. The semiconductor sensor is sensible to ethanol when the sensing part is heated to high temperature with an electronic resistor heater. However, the amount of power needed by the heated sensor (typically more than 50mW) could be excessive for certain low power applications, such as button-cell powered keychain or externally powered devices or power-harvesting devices.

The third method, using fuel-cell sensors, has the advantage to be both accurate and ultra-low power, as the fuel-cell element usually needs no heating elements. The sensing is performed by delivering a known volume of gas to a chamber which contains the fuel cell element and measuring the cell current output. As the fuel-cell output is dependent on the volume of gas flowed through the chamber, this has to be known. Methods for knowing *a priori* the volume consist in an active sampling system that pumps a fixed volume of gas inside the chamber with an electro-mechanical device. The power consumption and complexity of such system make it less effective for portable consumer devices if compared to other solutions.

Methods for knowing *a posteriori* the volume consists on a passive flow-meter which measures the volume passed through the chamber, and sometimes is called pump-less method. This method has been implemented sometimes with a thermal flow-meter. This has the issues of needing a certain amount of power for self-heating (typically more than 10mW), and generally has a poor response time and its accuracy depends on environmental conditions. This method has been implemented other times with a pressure-based flow-meter, with a calibrated orifice plate or a Venturi tube. These methods have the issues of being more complex to calibrate and less cost-effective.

An example of implementation of the third method, but related to spirometry measurement, is described in EP0437055, where a tube containing a diameter constriction is provided. The pressure difference between upstream and downstream tube is strictly dependent on fluid flow rate inside the tube. A differential pressure sensor is used to recover the flow measurement. To the skilled person it is immediately apparent that the problem of measuring a high volume of gas is quite different than measuring a small volume of gas, in which a small concentration of a specific substance must be detected and that the solution of the first problem is not applicable to the solution of the second one.

Another example of implementation of the third method is described in EP0834072. In the apparatus described, a pump is used to sample a predetermined gas volume from the main pipe while the user is blowing. This volume, when the pump is activated, passes through the chamber of a fuel-cell ethanol sensor with which the pump is in communication. This assures a known volume into the sensor, but the pump itself needs an amount of electrical current (typically more than 0.5A) which, even if needed impulsively, could be excessive for certain applications. Moreover, the implementation of such electro-mechanical pump could not be cost-effective for some applications.

Another example of implementation of the third method, which does not use a sampling pump, is also described in US6,026,674. The apparatus described uses a pressure sensor to detect the presence of breath flow and an electro-mechanical valve to deliver an amount of gas volume to the fuel-cell. In this implementation, the exact gas flow is not measured, but the dosing is demanded to the valve timing. Moreover, in this implementation, the power consumption of the electro-mechanical valve could be excessive for certain applications.

Another example of implementation of the third method, which does not use a sampling pump, is also described in US3,966,579. A fraction of the breath flow is passed through the fuel-cell and its short-circuit current is measured. However, a method to measure the sample volume in order to retrieve the concentration is not described.

Another example where a sampling pump is not used is described in US2011/0009765. The apparatus described uses a thermistor, possibly heated, to measure the flow rate in the main pipe and so to estimate the volume. The flow transfers heat to or from the thermistor, thus altering its temperature in a way that is dependent on the flow rate. Specific information on the accuracy of the flow measurement is not given in the document. Moreover, the power needed to heat the sensor could be excessive for certain applications.

An example of a method used for data processing inside an implementation of the third method is described in WO2012/087186, wherein the flow rate detected is continuously integrated to retrieve the total volume of sampled fluid, and this information is then used to compensate the signal from the ethanol sensor. The document aims to solve accuracy of the measurement and solves the problem through an improved mathematical method.

Power harvesting devices are devices that exploit a power source not intended by design to supply power to a device. Examples are moving objects or bodies and audio ports for earphones, headphones or loudspeakers.

An example of implementation of a power harvesting device using audio port is described in ACM DEV'10, December 17-18, 2010 (978-1-4503-0473-3-10/12), wherein a jack audio connector is connected to an electric transformer and a diode rectifier bridge, so that the alternating current (AC) voltage from the audio port is increased and converted to direct current (DC) voltage to power the device. In the document it is declared that the maximum power drawn from the audio port is less than 20 mW.

US4314564 discloses an apparatus for detecting gas concentration in exhaled breath of a subject comprising a sampling device, a detector and a display device. Further systems are disclosed in WO2012087187, US2013021153, GB2018596 and the work of Menz et al. (MENZ B: "Vortex flowmeter with enhanced accuracy and reliability by means of sensor fusion and self-validation", MEASUREMENT, INSTITUTE OF MEASUREMENT AND CONTROL. LONDON, GB, vol. 22, no. 3-4, 12 November 1997 (1997-11-12), pages 123-128, XP004123097).

There is still the need for an ultra-low power yet precise measurement system for gaseous ethanol concentration that can be cost-effective for a portable device, therefore based on a sampling system having the aforementioned advantages.

SUMMARY OF THE INVENTION

It has now been found a solution to the above-mentioned problem of providing a precise and accurate sampling system which needs ultra-low power consumption by using a vortex whistle flow meter as sampling system.

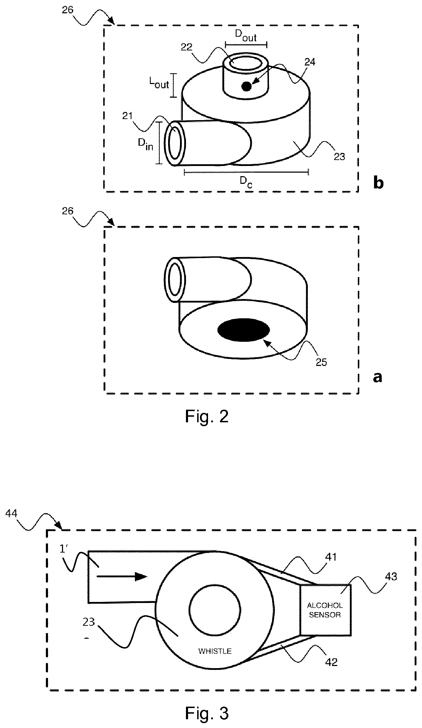

An example of implementation of the vortex whistle flow meter used in some of the embodiments of the present invention is described in US 2,794,341. The flow-meter is used for measuring air speed in airplanes, water speed in ships, pressure in tubes, or as a musical toy. In particular the apparatus described in FIG.2 of the mentioned document is suitable for the present invention.

The working principle of the vortex whistle flow meter (also known as vortex whistle) is described in the present document, but can be further understood in IEEE S 0018-9456(00)02236-1 (Sato, H. *et al.*) and in SICE TR 0007/99/3507-0840 (Sato, H. *et al.*).

An object of the present invention is an improved device for detecting and determining concentration of ethyl alcohol in a person's breath. The advantages of the invention are a very low-power consumption, low-cost and small size, but preserving a high accuracy on the sampled volume, resulting on an improved precision on the concentration measurement with respect to thermal flow-meter-based samplers. In terms of precision, the present invention can be related to samplers based on calibrated orifice or Venturi-tube, but presents many advantages in terms of construction simplicity and costs.

The present invention is applicable to a sensor system in all cases in which the sensor reading is dependent on the sampled volume, and so this volume has to be known.

It is an object of the present invention an apparatus comprising:

a. an assembly 15, wherein there is provided a main breath channel 1 for breath sampling, said main channel 1 is connected in parallel with a secondary channel 2, so that a portion of the main flow of the sampled breath passes through the secondary channel 2 and returns to the main channel;
b. an ethanol sensor 3 placed in the secondary channel, so that said portion of the sampled breath passes through said sensor, said sensor can optionally be equipped with a temperature sensor;
c. a vortex whistle flow-meter 5 placed at the end of the main channel 1 to collect the sampled breath;
d. a transducer 7 located in the vortex whistle flow meter 5 to obtain an electrical signal;
e. two amplifiers 8, 9 connected by way of input to the sensor 3 and to the transducer 7, respectively and by way of output to a microprocessor with a memory containing a software algorithm 10 for the calculation of ethyl alcohol concentration;

f. a display 12 connected to the microprocessor 10.

Within the meanings of the present invention, said apparatus is also briefly named "breath analyser".

Advantageously, said apparatus can be portable or fixed.

In a preferred embodiment, in the apparatus according to the present invention said detected ethanol concentration is directly correlated to ethanol blood concentration in said subject.

In a preferred embodiment of the present invention, breath or blood ethanol concentration in said subject is displayed.

Alternatively, instead of the item a) above, in said assembly **15** there is provided: a'. a main breath channel **1** for breath sampling, said main channel **1** is connected in series with said vortex whistle flow meter **23,** from which a first tangential pipe **41** connected to the said vortex whistle flow meter **23** and to the sensor **43,** and then reconnected to the said vortex whistle flow meter **23** by a second tangential pipe **42.**

In different embodiments of the present invention, in said apparatus said transducer **7** is a microphone or a piezoelectric transducer.

In the apparatus according to the present invention, power is provided by at least one battery or by an external power source. Therefore, a power channel **11** is provided to supply the necessary power to the device. Conveniently, the power channel **11** can also be a communication channel to receive and transmit data to and from the device, for example in case of a wired channel. Channel **11** can be a communication channel only. For example, in case of wireless, the communication channel **11** is coupled with a power supply. Also in case of a wired communication channel, an external power supply should be provided.

In a preferred embodiment, in said apparatus, said communication and power channel **11** is a Universal Serial Bus connection, preferably said communication channel **11** is a wireless connection for communication and a battery for power.

In a more preferred embodiment, in said apparatus said communication and power channel **11** is an audio jack connector.

In another embodiment, said communication channel is a jack connector and power is supplied by a separate battery.

In another preferred embodiment, said apparatus further comprises an intelligent device with display **13** connected to the microprocessor **10** instead of the display **12.**

Said intelligent device **13** is selected from the group consisting of a smartphone, a laptop and a desktop personal computer.

In another preferred embodiment, said apparatus further comprises a vehicle interlock system **14** connected to said microprocessor **10.**

In a preferred embodiment, said apparatus is connected with said intelligent device **13** or said vehicle interlock system **14** with a data communication channel **11,** both in a wired or wireless mode.

It is also herein disclosed a method for detecting breath alcohol concentration (BrAC) and subsequently, if desired, converting said blood alcohol concentration (BAC) in a subject comprising sampling a subject's breath into the above apparatus.

According to this method, the exhaled breath sampled from said subject produces an acoustic wave in said vortex whistle flow meter, said acoustic wave having a frequency $f$ and the method comprises:

    a. converting said frequency $f$ into a flow rate $F;$
    b. calculating from said flow rate $F$ the total volume $V$ of said sampled breath;
    c. integrating current $I$ from said detector to obtain the total charge $Q;$
    d. calculating BrAC according to equation (4)

$$BrAC = K \cdot \frac{Q}{V}$$

wherein K is a calibration parameter;
    e. optionally converting BrAC into BAC according to equation (6).

$$BAC = K' \cdot BrAC$$

The above mentioned apparatus can be incorporated in a vehicle interlock system **14,** which can be mounted on a vehicle. Both the system and the vehicle are objects of the present invention, together with a method for controlling the operation of a vehicle provided with said vehicle interlock system **14** in function of the blood alcohol concentration of the vehicle operator, said method comprising:

a. determining blood alcohol concentration of said vehicle operator, by sampling said operator's breath into said system;
b. comparing said blood alcohol concentration with a determined alcohol concentration set into said interlock system;
c. operating said interlock system, in order to prevent the user to ignite the vehicle, if said blood alcohol concentration is equal or higher than said determined alcohol concentration.

A method for operating the apparatus herein disclosed is another object of the present invention and said method comprises:

a. placing the apparatus in the START condition **51;**
b. providing an exhaled breath sample for a certain amount of time, in which sampler data, in form of the detected frequency $f$ are collected **52;**
c. converting frequency $f$ into flow rate $F$ by inverting equation (1) $f = k \cdot F$;
d. passing said apparatus to a decisional condition **53** in which, if the flow F is greater than a predefined threshold $F_2$, the apparatus passes to the subsequent condition **54,** otherwise it goes back to condition **52;**
e. in the condition **54,** both the vortex whistle flow meter sampler and the sensor are sampled for a certain amount of time;
f. passing said apparatus to condition **55** in which the acquired flow data is integrated to obtain the total volume data according to the equation (2)

$$V(nT) = \sum_{k=1}^{n} F(kT) \cdot T$$

in which $F(kT)$ is the flow value recovered with equation (1) from the frequency $f$ measured in the $k\text{-}th$ sampling window, $T$ is the length of the sampling window, $V(nT)$ is the volume summed from the first to the n-th sampling window, and the first sampling window is taken when the condition **53** is first verified, and so determines the beginning of the flow;
g. passing said apparatus to decisional condition **56** in which, if the volume $V$ is greater than a predefined threshold $V_1$, the apparatus passes to the condition **58,** otherwise it goes to the condition **57.** In this decisional condition, if the flow $F$ is greater than a predefined threshold $F_1$ the apparatus goes back to the condition **54** in which it continues to sample and integrate the two values, otherwise it passes to the FAIL condition **62;**
h. in the condition **58,** acquiring detector current $I$ and integrating it to obtain the total charge $Q$ according to equation (3)

$$Q(nT) = \sum_{k=1}^{n} I(kT) \cdot T$$

in which $I(kT)$ is the sensor current measured and optionally averaged in the k-th sampling window, $T$ is the length of the sampling window, $Q(nT)$ is the charge summed from the first to the n-th sampling window, and the first sampling window is taken when the condition **53** is first verified, and so determines the beginning of the flow;
i. passing said apparatus to condition **59** in which the BrAC is calculated according to equation (4)

$$BrAC = K \cdot \frac{Q}{V}$$

in which K is a calibration parameter, Q is the charge obtained from equation (3) and V is the volume obtained from equation (2); the apparatus then passes to the condition **60** in which the BrAC result is displayed, or transmitted or stored for further usage; the measurement process then concludes to the END condition **61;**
j. displaying BrAC.

The present invention will be now disclosed in detail also by means of drawings and examples.

BRIEF DESCRIPTION OF THE DRAWINGS

While the following paragraphs will describe the invention in details, it is believed that such details can be better understood with the aid of the drawings in which:

Fig. 1 shows the setup of a breath ethanol concentration detector device according to the first embodiment of the invention, considering only some elements, or to the fourth or fifth embodiments of the invention, considering different elements;
Fig. 2 shows in detail the shape of the vortex whistle flow meter as long as the positioning of the microphone and the piezoelectric transducer;
Fig. 3 shows the tangential air dump system according to the second embodiment of the invention;
Fig. 4 shows a flow chart of the measurement method for an apparatus including an intelligent device with display, according to the fourth embodiment of the invention, or for an apparatus according to the fifth embodiment of the invention.
Fig. 5 shows a flow chart of another measurement method for an apparatus including an intelligent device with display, according to the fourth embodiment of the invention, or for an apparatus according to the fifth embodiment of the invention.

DETAILED DESCRIPTION OF THE INVENTION

The present invention is now disclosed in detail through some preferred embodiments. In this connection, the embodiments are provided in the exemplary working of the invention for the detection of ethyl alcohol concentration in the exhaled breath of a subject and the subsequent determination of his/her ethyl alcohol concentration in blood. As an exemplary embodiment, the detection of ethyl alcohol is made by means of a fuel-cell sensor.

For the purposes of the present invention the terms "system", "device" and "apparatus" are meant as synonyms.

For the purposes of the present invention the terms "ethyl alcohol", "ethanol" and "alcohol" are meant as synonyms.

For the purposes of the present invention the terms "user", "subject", and "person" are meant as synonyms.

When a breath sample is passed through the chamber of a fuel-cell ethanol sensor, an electrochemical reaction takes place together with an electrolyte solution inside the sensor, and an electric current can be measured at the sensor electrodes.

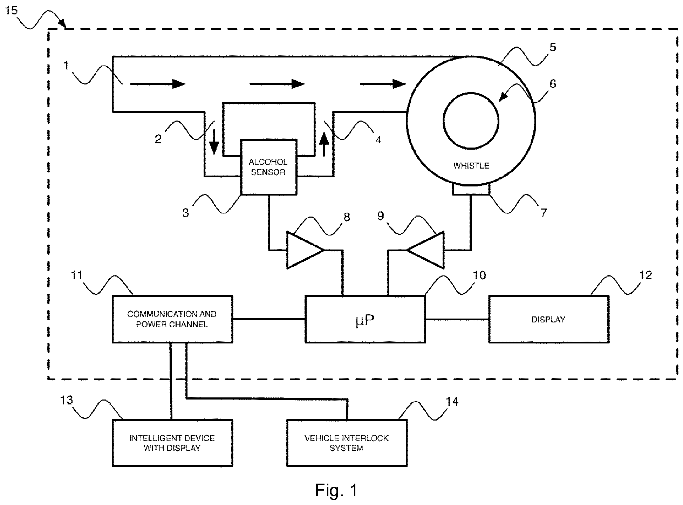

According to a first embodiment of the invention (see Fig. 1) in the above apparatus, there is provided an assembly **15,** comprising a sampling device (also called whistle), a detector **3** (also called sensor, and for this specific first embodiment of the invention is an ethyl alcohol sensor), and a display device **12** is shown. In the sampling device, there is provided a breath channel **1,** called the main breath channel. The arrows show the flow direction of the sampled breath from the subject whose blood alcohol concentration is to be measured. The main breath channel **1** is connected in parallel with a secondary channel **2,4.** A portion of the main flow passes through the secondary channel **2** and returns to the main channel. An ethanol sensor **3,** for example a fuel-cell sensor, is placed in the secondary channel. A vortex whistle flow meter **5** is placed at the end of the main channel **1.** The vortex whistle flow meter **5** is composed of a cylindrical chamber with a tangential inlet and an axial outlet **6,** such as for example shown in the above mentioned US 2,794,341, FIG. 2. A microphone or piezoelectric transducer **7** is located in the vortex whistle flow meter **5** to obtain an electrical signal. Two amplifiers **8, 9** are connected by way of input one to the sensor **3** and one to the microphone or transducer **7** and by way of output to a microprocessor with a memory containing a software algorithm **10** for signal processing and the calculation of ethyl alcohol concentration. A display **12** is connected to the microprocessor **10.**

The system according to the present invention can optionally be connected to an intelligent device with display **13,** such as for example a smart-phone, instead of the display **12.**

The system according to the present invention can optionally be connected to a vehicle interlock system **14.**

In a second embodiment of the invention, as far as the vortex whistle flow meter is concerned, and as shown in Fig. 3 the secondary flow is obtained in an alternative way, by means of two tangential pipes connected to the vortex flow chamber. The two pipes are then connected to the ethanol sensor.

In a third embodiment of the invention, a data communication channel, wired or wireless is added to the microprocessor system.

In a fourth embodiment of the invention, a data communication channel connected to the microprocessor system and to an intelligent device (smart-phone) with display and user interface is added to the system. The raw flow and current data and/or the calculated measurement are transmitted to the intelligent display device. The smart-phone can optionally execute an application that communicates with the device in order to display, elaborate and share the data collected from the device. The application can optionally represent on the smartphone screen the BAC and/or BrAC measured.

The application can optionally calculate and display the "recovery time", which is the time by which the user's BAC is expected to fall below a certain selected limit. The calculation can be possibly made accounting for the user's physical characteristics. Examples of these implementations can be found on applications such as "AlcoDroid Alcohol Tracker" by Myrecek or "DrinkTracker" by slappmedotcom Pty Ltd.

In a fifth embodiment of the invention, a data communication channel connected to the microprocessor system and to an interlock system for a vehicle is added to the system.

Reference will now be made in detail to the present exemplary embodiments of the invention, examples of which are illustrated in the drawings, in which each number indicates a unique element.

Referring to Fig. 1, the assembly **15** has a main flow channel made of a main pipe **1** with an inlet for receiving the user's breath. The end of the main pipe is connected to the inlet of a vortex whistle flow-meter **5** which has an outlet **6** to allow the flow to be expelled. The main pipe has a lateral derivation **2** connected to an ethanol sensor **3** which is then connected to another derivation **4** that reconnects to the main pipe. In this way, the airflow inside the sensor is proportional to the flow in the main pipe. The ethanol sensor output is an electrical current. The output of the vortex whistle flow meter is an electrical voltage audio signal delivered by an acoustic transducer **7**. The output of the ethanol sensor and the whistle are electrical signals and are connected to two amplifiers **8** and **9** that are then connected to a microprocessor unit with memory **10**.

Referring to Fig. 2a,b, the vortex whistle flow meter **26** is provided with a tangential inlet **21,** a round chamber **23** and an axial outlet **22**. The airflow entering into the chamber produces an instable fluid dynamic condition that results in an oscillation which frequency *f* is linearly proportional to the input flow *F* according to equation (1).

$$ f = k \cdot F \qquad\qquad (11) $$

wherein *k* is a calibration constant which is stored for example in the microprocessor memory.

The oscillation produces an acoustic wave at the same frequency. The acoustic wave can be measured with the transducer **7.** In an exemplary embodiment of the invention, the transducer **7** is a microphone **24** positioned on the axial outlet whereas in another exemplary embodiment it is a piezoelectric transducer **25** positioned on the bottom wall, either on the outside or on the inside face.

Always referring to Fig. 2b, it has been found that a determined interval of mechanical dimensions of the vortex whistle flow meter **26** makes it compatible with the dynamic range of the flow rate of the user's blowing (5-15 L/min). Such dimensions grant a good signal-to-noise ratio (SNR) over the said dynamic range and mitigate intrinsic nonlinearities. In a preferred embodiment of the vortex whistle flow meter **26** such dimensions are:

- $D_{in}$ from 4 to 7mm,
- $D_{out}$ from 6 to 9mm,
- $D_c$ from 25 to 35mm;
- $L_{out}$ from 4 to 15mm;

wherein $D_{in}$ represents the diameter of the tangential inlet **21,** $D_{out}$ represents the diameter of the axial outlet **22,** $D_c$ represents the diameter of the chamber **23** and $L_{out}$ represents the length of the axial outlet **22.**

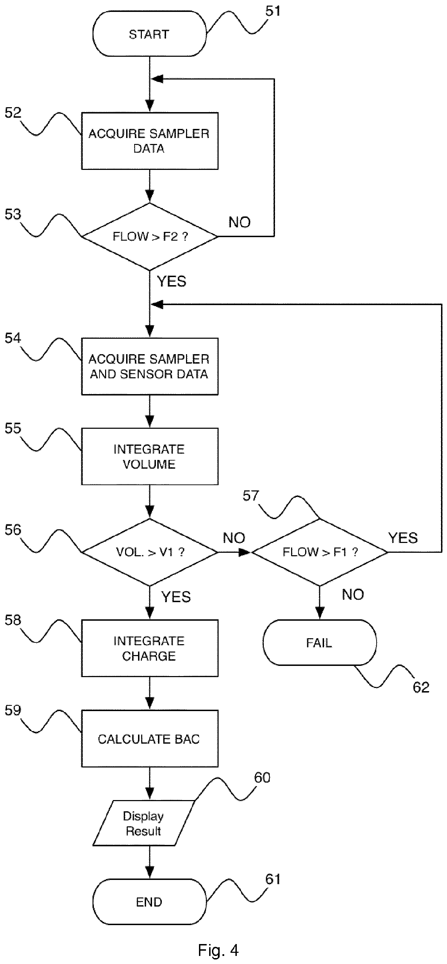

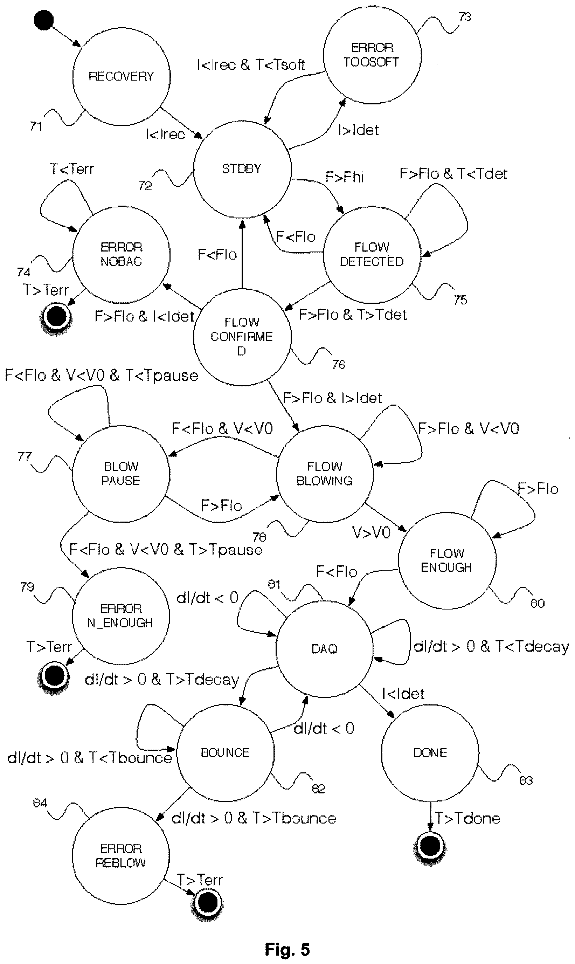

In the first embodiment of the invention, the microprocessor is connected to a display **12**. The microprocessor **10** loads the application code, made according to the method described in Fig. 4 or Fig. 5, from its memory. The signal from the amplifier **9** connected to the flow-meter is pre-elaborated by the microprocessor in order to recover the frequency *f,* which is then used as the first input for the algorithm. The signal from the amplifier **8** connected to the ethanol sensor is used to recover the sensor current *I*, which is used as the second input for the algorithm. After performing the algorithm, the calculated *BrAC* (see equation (4) below, or an equivalent algorithm, such as for example WO 2012/087186) or BAC (see equation (5) below)result is displayed on the display **12,** which is connected to the microprocessor.

Optionally, in the case of embodiments comprising an intelligent display device (fourth) or vehicle interlock (fifth) the algorithm in Fig. 4 or Fig. 5 can be implemented totally on these devices and the microprocessor only instructed to acquire data and perform signal processing to recover *f.* Each intermediate solution, where parts of the algorithms in Fig. 4 or Fig. 5 are implemented on the microprocessor and complementary parts implemented on the intelligent devices, are also comprised in the present invention.

In one exemplary embodiment of the invention the assembly **15** can be powered by batteries or external power.

In a second embodiment of the invention, the assembly **15** is modified in the components **2, 4, 5,** shown in Fig. 1, according to the setup **44** described in Fig. 3. The lateral derivation (i.e. the secondary channel) is not realized on the main pipe but on the vortex chamber **23**. A first tangential pipe **41** is connected to the vortex chamber and to the sensor **43,** and is then reconnected to the chamber by a second tangential pipe **42**. In this way part of the airflow is spilled out of the chamber and then back injected in it. In an opportune interval, the flow passing through the sensor is proportional to the flow entering in the device. This provides the same performance of the first embodiment of the invention, but reducing the occupied space.

In the third embodiment of the invention, the communication and power channel **11** is used both for powering the device and transfer the acquired or calculated data from the method in Fig. 4 or Fig. 5. In an exemplary embodiment of the invention, the communication channel and power channel **11** is a Universal Serial Bus (USB) connection. In another exemplary embodiment of the invention, the channel **11** can be a wireless connection for communication and a battery for power. Yet in another exemplary embodiment of the invention, the communication channel and power channel **11** is a 4-way audio jack connector, in which a stereo earphone line and a microphone line are available. The microphone line is used for communicating data from the assembly **15** to the device and earphones line is used for harvesting/receiving power from the device which the assembly **15** is connected to.

In the fourth embodiment of the invention, the intelligent device with display **13** is connected to the assembly **15** through the communication and power channel **11**. In an exemplary embodiment of the invention, the device **13** is a smartphone or tablet, whereas in another embodiment it is a laptop, desktop pc. This allows the algorithm implementing the method represented in Fig. 4 or Fig. 5 to be executed into the intelligent device. The acquired data regarding the frequency *f* and the current *I* are transmitted directly to the device **13** and then elaborated with the method in Fig. 4 or Fig. 5.

In the fifth embodiment of the invention, the vehicle interlock system **14** is connected to the assembly **15** through the communication and power channel **11**. After performing the measurement and having it sent to the interlock system, the interlock system can decide whether to allow the user to ignite the vehicle or not, according to the data received.

In an exemplary embodiment of the invention, the device **14** is provided with an intelligent microprocessor system. This allows the algorithm implementing the method represented in Fig. 4 or Fig. 5 to be executed into the intelligent device. The acquired data regarding the frequency *f* and the current *I* are transmitted directly to the device **14** and then elaborated with the method in Fig. 4 or Fig. 5.

Referring to Fig. 4, the implemented method makes the device to be in the START condition **51** after powering it on, either by means of a power switch or by connecting it to the devices **13** or **14**. From this condition, the system passes to the condition **52** for a determined amount of time (called the sampling window), in which it samples the sampler data, in form of the detected frequency *f*. The amount of time spent in each sampling window depends on the resolution with which the condition **53** has to be evaluated. In the present device, sampling windows between 50ms and 500ms can be used. The frequency *f* is converted to the flow rate *F* inverting equation (1). Then the system passes to the decisional condition **53** in which, if the flow *F* is greater than a predefined threshold $F_2$, the system passes to the condition **54,** otherwise it goes back to the condition **52**. In the condition **54,** both the vortex whistle flow meter sampler and the sensor are sampled for the time of the sampling window. Then the system passes to the condition **55** in which the acquired flow data is integrated to obtain the total volume data according to the equation (2).

$$V(nT) = \sum_{k=1}^{n} F(kT) \cdot T \qquad (2)$$

In which *F(kT)* is the flow value recovered with equation (1) from the frequency *f* measured in the *k-th* sampling window, *T* is the length of the sampling window, *V(nT)* is the volume summed from the first to the n-th sampling window, and the first sampling window is taken when the condition **53** is first verified, and so determines the beginning of the flow.

Then the system passes to the decisional condition **56** in which, if the volume *V* is greater than a predefined threshold $V_1$, the system passes to the condition **58,** otherwise it goes to the condition **57.** In this decisional condition, if the flow *F* is greater than a predefined threshold $F_1$ the system goes back to the condition **54** in which it continues to sample and integrate the two values, otherwise it passes to the FAIL condition **62** in which the measurement has failed as the user stopped blowing and the sampled volume is not enough to perform the measurement.

In the condition **58**, also the acquired sensor current *I* is integrated to obtain the total charge *Q* thanks to the equation (3).

$$Q(nT) = \sum_{k=1}^{n} I(kT) \cdot T \qquad (3)$$

In which *I(kT)* is the sensor current measured and optionally averaged in the k-th sampling window, *T* is the length of the sampling window, *Q(nT)* is the charge summed from the first to the n-th sampling window, and the first sampling window is taken when the condition **53** is first verified, and so determines the beginning of the flow.

The system then passes to the condition **59** in which the BrAC is calculated on the basis of the acquired data, for example the BrAC is obtained with equation (4), which accounts for the count of ethyl alcohol quantity in the given volume.

$$BrAC = K \cdot \frac{Q}{V} \qquad (4)$$

In which $K$ is a calibration parameter, $Q$ the charge obtained from equation (3) and $V$ is the volume obtained from equation (2).The system then passes to the condition **60** in which the BrAC result is displayed, or transmitted or stored for further usage. The measurement process then concludes to the END **61.** A temperature sensor can be added to the system and equation (4) is improved according to the variation of the sensor conversion efficiency to operating temperature, resulting in equation (5).

$$BrAC = K(T_{sens}) \cdot \frac{Q}{V} \qquad (5)$$

wherein $K(T_{sens})$ is a function of the sensor temperature $T_{sens}$ and $K(T_{sens})$ is obtained by interpolation of calibration points measured at different temperatures;

In an exemplary embodiment of the invention, together with the BrAC, also the BAC is displayed at the end of the measurement, and is calculated thanks to the well-known relation (6)

$$BAC = K' \cdot BrAC \qquad (6)$$

In which K' is a constant which varies from 2100 to 2300, depending on the subject and on the drinking time.

In another embodiment of the invention, after terminating the execution, the system goes back to the START condition **51** for a new measurement.

In the following, each time the flow value $F$ is mentioned, it is implicitly meant that the sampler data is acquired for the above-mentioned sampling window and the flow $F$ is recovered from the measured frequency $f$.

Typical values considered for the thresholds are: $F_1$ = 10L/min, $F_2$ = 11L/min, $V_1$ = 1.5L.

Referring to Fig. 5, the implemented method makes the device to be in the RECOVERY condition **71** after powering it on, either by means of a power switch or by connecting it to the devices **13** or **14.** From this condition, when the sensor current $I$ is less than a determined constant $I_{rec}$ the system passes to the STDBY status **72.** From this condition, if the sensor current $I$ goes higher than the constant $I_{det}$ the system passes to the ERROR TOOSOFT status **73,** because it is likely that a weak breath was sufficient to determine a sensor current but not a flow measurement. From the STDBY status **72,** if the detected flow is greater than $F_{hi}$, the system passes to the FLOW DETECTED status **75.** In this condition, flow and current are integrated according to equations (2) and (3). From this condition, if the flow remains greater than $F_{lo}$ for a time $T_{det}$, the system passes to the FLOW CONFIRMED status **76** in which the integration is continued, or, in case the detected flow is less than $F_{lo}$, the system goes back to the STDBY status **72.** From the FLOW CONFIRMED status **76,** if the detected flow is greater than $F_{lo}$ and the sensor current is less than to $I_{det}$, the system passes to the ERROR NOBAC status **74,** which means that either the flow ethanol concentration is less than value associated to $I_{det}$, or that the measured flow was due not to an actual flow but to an interference; or to the FLOWBLOWING status **78,** in which the integration is continued. From the ERROR NOBAC status **74,** after the time $T_{err}$, the system terminates the execution. Alternatively, if also the current is above $I_{det}$, the system recognizes actual flow and goes on with the measurement process, which means that the user is breathing inside the device. From the FLOW BLOWING status **78,** the system passes to the BLOW PAUSE status **77** if the flow is less than $F_{lo}$, as the user could stop blowing for a while; or to the state FLOW ENOUGH if the integrated volume $V$ is greater than $V_0$, which means that the blown volume is sufficient to perform the measurement. In the FLOW ENOUGH state **80** the integration is continued. From the BLOW PAUSE status **77,** the system goes back to the FLOW BLOWING status **78** if the flow is greater than $F_{lo}$, or to the ERROR N_ENOUGH status **79** if the flow remains lower than $F_{lo}$ for at least the time $T_{pause}$, which means that the blown volume is not enough to perform the measurement. From the FLOW ENOUGH status **80,** the system passes to the DAQ state **81** if the flow is lower than $F_{lo}$. In this condition, the system has received a sufficient and acceptable amount of volume and is waiting for the sensor current $I$ to terminate its transient. From the ERROR N_ENOUGH status **79,** after the time $T_{err}$, the system terminates the execution. From the DAQ status **81,** the system passes to the DONE status **83** if the current becomes lower than $I_{det}$, or to the BOUNCE status **82** if the current is increasing after a timeout $T_{decay}$ from when the system passes for the first time to the DAQ status **81.** This condition is provided to tolerate the decay response time and little current peaks over a general decay behaviour. From the BOUNCE status **82** the system passes to the ERROR REBLOW status **84** if the current is increasing for a period greater than $T_{bounce}$, or back to the DAQ status **81** if the current is increasing. In the DONE status **83** the BAC and/or the BrAC are calculated according to the equation (4) or (5) and (6)and after the time $T_{done}$, the system terminates the execution. From the ERROR REBLOW status **84,** after the time $T_{err}$, the system terminates the execution.

In another embodiment of the invention, after terminating the execution, the system goes back to the RECOVERY status **71** for a new measurement.

The aforementioned parameters are:

- $F(t_i)$ is the flow value measured in the sampling window $t_i$, $\Delta t_i$ is the length of the sampling window $t_i$;
- $I(t_i)$ is the sensor current measured and optionally averaged in the sampling window $t_i$;
- V(tk) is the volume summed from the instant $t_0$ to the instant $t_k$;
- $F_{lo}$ is an appropriate threshold for the flow to establish that the detected flow is sufficient to begin the measurement, i.e. 10 L/min;
- $F_{hi}$ is an appropriate threshold for the flow to establish whether the flow remains sufficient to continue the measurement, i.e. 11 L/min;
- $I_{rec}$ is an appropriate threshold for the current to establish that the sensor functionality is recovered, i.e. 15µA;
- $I_{det}$ is an appropriate threshold for the current to establish that a minimum alcohol presence is detected, i.e. 25µA;
- $V_0$ is an appropriate threshold for the volume to establish that a sufficient volume has been sampled to perform the measurement, i.e. 2 L;
- $T_{pause}$ is an appropriate timeout to allow the user to stop breathing for a while, i.e. 0.5 s;
- $T_{det}$ is an appropriate timeout to establish whether a sufficient flow is been blown constantly or impulsively, i.e. 2 s;
- $T_{decay}$ is an appropriate timeout to allow the sensor current not to begin decaying right after the flow stops, i.e. 2 s;
- $T_{bounce}$ is an appropriate timeout to filter sensor current bounces during decay, i.e. 0.2 s;
- $T_{done}$ is an appropriate timeout to display result and after return to recovery status, i.e. 3 s;
- $T_{err}$ is an appropriate timeout to display error and after return to recovery status, i.e. 3 s:

The $F_{hi}$ and $F_{lo}$ threshold values are generally different in order to grant a hysteretic behaviour.

The implemented method allows accounting for device misuse such as intermittent flow, weak flow, repeated or too strong flow, that could seriously affect both accuracy and precision of the measurements.

By the term "appropriate", it is intended that the appropriate values can be set by the manufacturer in order to bring the device in compliance with the local laws concerning the ethanol levels (BAC) in order to determine a person's sobriety. In the specific embodiment of the present invention concerning a vehicle interlock system, the appropriate values can be set in order to determine start of the vehicle or any other means allowing or not the operation of the vehicle.

In other embodiments of the present invention, the appropriate values, when referring to specific gaseous substances to be measured in a person's breath, can be set in order to comply for predetermined levels of said substance, for example referring to clinical parameters.

## Claims

1. An apparatus for detecting ethanol concentration in exhaled breath of a subject, comprising

   a. an assembly (**15**), wherein there is provided a main breath channel (**1**) for breath sampling, said main channel (**1**) is connected in parallel with a secondary channel (**2**), so that a portion of the main flow of the sampled breath passes through the secondary channel (**2**) and returns to the main channel;
   b. an ethanol sensor (**3**) placed in the secondary channel, so that said portion of the sampled breath passes through said sensor; said sensor (**3**) optionally comprising a temperature sensor;
   c. a vortex whistle flow-meter (**5**) placed at the end of the main channel (**1**) to collect the sampled breath;
   d. a transducer (**7**) located in the vortex flow-meter (**5**) to obtain an electrical signal;
   e. two amplifiers (**8**), (**9**) connected by way of input to the sensor (**3**) and to the transducer (**7**), respectively and by way of output to a microprocessor with a memory containing a software algorithm (**10**) for the calculation of ethyl alcohol concentration;
   f. a display (**12**) connected to the microprocessor (**10**).

2. The apparatus according to claim 1, wherein said apparatus is portable or fixed.

3. The apparatus according to claim 1 or 2, wherein said detected ethanol concentration is directly correlated to ethanol blood concentration in said subject.

4. The apparatus according to claim 3, wherein ethanol breath or said ethanol blood concentration in said subject is displayed.

5. The apparatus according to any one of claims 1-4, wherein said ethanol sensor (**3**) is a fuel-cell sensor.

6. The apparatus according to claim 1, wherein in said assembly (**15**), in alternative to item a. there is provided:
   a'. a main breath channel (**1**) for breath sampling, said main channel (**1**) is connected in series with said vortex whistle flow-meter (**23**), from which a first tangential pipe (**41**) connected to the said vortex whistle flow-meter (**23**) and to the sensor (**43**), and then reconnected to the said vortex whistle flow-meter (**23**) by a second tangential pipe (**42**).

7. The apparatus according to any one of claims 1-6, wherein said transducer (**7**) is a microphone or a piezoelectric transducer.

8. The apparatus according to any one of claims 1-7, wherein said vortex whistle flow-meter has the following dimensions:

   • $D_{in}$ from 4 to 7mm,
   • $D_{out}$ from 6 to 9mm,
   • $D_c$ from 25 to 35mm;
   • $L_{out}$ from 4 to 15mm;

   wherein $D_{in}$ represents the diameter of the tangential inlet (**21**), $D_{out}$ represents the diameter of the axial outlet (**22**), $D_c$ represents the diameter of the chamber (**23**) and $L_{out}$ represents the length of the axial outlet (**22**).

9. The apparatus according to any one of claims 1-8, wherein power is provided by at least one battery or by an external power source through a power channel (**11**).

10. The apparatus according to claim 9, wherein said power channel (**11**) is also a communication and power channel or only a communication channel.

11. The apparatus according to claim 10, wherein said communication and power channel (**11**) is a Universal Serial Bus connection.

12. The apparatus according to any one of claims 1-10, wherein said communication channel (**11**) is a wireless connection for communication and a battery for power.

13. The apparatus according to any one of claims 1-10, wherein said communication channel (**11**) is an audio jack connector and a battery for power.

14. The apparatus according to any one of claims 1-10, wherein said communication and power channel (**11**) is an audio jack connector.

15. The apparatus according to any one of claims 1-14, further comprising an intelligent device with display (**13**) connected to the microprocessor (**10**) instead of the display (**12**).

16. The apparatus according to claim 15, wherein said intelligent device (**13**) is selected from the group consisting of a smartphone, a laptop and a desktop personal computer.

17. The apparatus according to any one of claims 1-16, further comprising a vehicle interlock system (**14**) connected to said microprocessor (**10**).

18. The apparatus according to any one of claims 15-16 or claim 17, wherein the connection with said intelligent device (**13**) or said vehicle interlock system (**14**) is a data communication channel (**11**).

19. The apparatus according to claim 18, wherein said data communication channel (**11**) is wired or wireless.

20. A vehicle comprising the apparatus of any one of claims 17-19.

21. Method for controlling the operation of the vehicle of claim 20 in function of the blood alcohol concentration of the vehicle operator, comprising:

   a. determining blood alcohol concentration of said vehicle operator, by sampling said operator's breath into the apparatus of any one of claims 17-19;
   b. comparing said blood alcohol concentration with a determined alcohol concentration set into said interlock system (**14**);
c. operating said interlock system (**14**), in order to prevent the user to ignite the vehicle, if said blood alcohol concentration is equal or higher than said determined alcohol concentration.

**22.** Method for operating the apparatus of any one of claims 1-16, comprising:

a. placing the apparatus in the START condition (**51**);
b. providing an exhaled breath sample for a certain amount of time, in which sampler data, in form of the detected frequency *f* are collected;
c. converting frequency *f* into flow rate *F* by inverting equation $f = k \cdot F$;
d. passing said apparatus to a decisional condition (**53**) in which, if the flow F is greater than a predefined threshold **F2,** the apparatus passes to the subsequent condition (**54**), otherwise it goes back to condition (**52**);
e. in the condition (**54**), both the vortex flow-meter sampler and the sensor are sampled for a certain amount of time;
f. passing said apparatus to condition (**55**) in which the acquired flow data is integrated to obtain the total volume data according to the equation (2)

$$V(nT) = \sum_{k=1}^{n} F(kT) \cdot T$$

in which *F(kT)* is the flow value recovered with equation (1) from the frequency *f* measured in the *k-th* sampling window, *T* is the length of the sampling window, *V(nT)* is the volume summed from the first to the n-th sampling window, and the first sampling window is taken when the condition (**53**) is first verified, and so determines the beginning of the flow;
g. passing said apparatus to decisional condition (**56**) in which, if the volume *V* is greater than a predefined threshold $V_1$, the apparatus passes to the condition (**58**), otherwise it goes to the condition (**57**);in this decisional condition, if the flow F is greater than a predefined threshold **F1** the apparatus goes back to the condition (**54**) in which it continues to sample and integrate the two values, otherwise it passes to the FAIL condition (**62**);
h. in the condition (**58**), acquiring sensor current *I* and integrating it to obtain the total charge *Q* according to equation (3)

$$Q(nT) = \sum_{k=1}^{n} I(kT) \cdot T$$

in which *I(kT)* is the sensor current measured and optionally averaged in the k-th sampling window, *T* is the length of the sampling window, *Q(nT)* is the charge summed from the first to the n-th sampling window, and the first sampling window is taken when the condition (**53**) is first verified, and so determines the beginning of the flow;
i. passing said apparatus to condition (**59**) in which the BrAC is calculated according to equation (4)

$$BrAC = K \cdot \frac{Q}{V}$$

in which K is a calibration parameter, Q is the charge obtained from equation (3) and V is the volume obtained from equation (2), the apparatus then passes to the condition (**60**) in which the BrAC result is displayed, or transmitted or stored for further usage, the measurement process then concludes to the END (**61**);
j. displaying BrAC;
k. optionally converting breath alcohol concentration (BrAC) into blood alcohol concentration (BAC) and displaying said BAC.

**23.** Method according to claim 22, further comprising:

l. calculating and displaying the recovery time.

**Patentansprüche**

**1.** Vorrichtung zum Detektieren einer Ethanolkonzentration in ausgeatmeter Atemluft eines Probanden, die Folgendes umfasst:

a. eine Baugruppe (15), in der ein Haupt-Atemluftkanal (1) zur Atemluft-Probennahme vorhanden ist, wobei der Hauptkanal (1) mit einem sekundären Kanal (2) parallel so verbunden ist, dass ein Teil des Hauptstromes der als Probe genommenen Atemluft durch den sekundären Kanal (2) strömt und zu dem Hauptkanal zurückkehrt;

b. einen Ethanolsensor (3), der in dem sekundären Kanal so angeordnet ist, dass der Teil der als Probe genommenen Atemluft durch den Sensor strömt; wobei der Sensor (3) optional einen Temperatursensor umfasst;

c. einen Wirbelpfeifen-Strömungsmesser (5), der am Ende des Hauptkanals (1) angeordnet ist, um die als Probe genommene Atemluft aufzufangen;

d. einen Messwertwandler (7), der in dem Wirbelströmungsmesser (5) angeordnet ist, um ein elektrisches Signal zu erhalten;

e. zwei Verstärker (8), (9), die mittels eines Eingangs mit dem Sensor (3) bzw. dem Messwertwandler (7) verbunden sind und mittels eines Ausgangs mit einem Mikroprozessor verbunden sind, der einen Speicher aufweist, der einen Software-Algorithmus (10) für die Berechnung einer Ethylalkohol-Konzentration enthält;

f. ein Display (12), das mit dem Mikroprozessor (10) verbunden ist.

2. Vorrichtung nach Anspruch 1, wobei die Vorrichtung transportabel oder ortsfest ist.

3. Vorrichtung nach Anspruch 1 oder 2, wobei die detektierte Ethanolkonzentration direkt mit der Ethanol-Blut-Konzentration des Probanden korreliert ist.

4. Vorrichtung nach Anspruch 3, wobei der Ethanol-Atem oder die Ethanol-Blut-Konzentration des Probanden angezeigt wird.

5. Vorrichtung nach einem der Ansprüche 1-4, wobei der Ethanolsensor (3) ein Brennstoffzellensensor ist.

6. Vorrichtung nach Anspruch 1, wobei in der Baugruppe (15), alternativ zu Punkt a., Folgendes vorhanden ist:

a'. ein Haupt-Atemluftkanal (1) zur Atemluft-Probennahme, wobei der Hauptkanal (1) in Reihe mit dem Wirbelpfeifen-Strömungsmesser (23) verbunden ist, von dem aus ein erstes Tangentialrohr (41) mit dem Wirbelpfeifen-Strömungsmesser (23) und mit dem Sensor (43) verbunden ist und dann erneut mit dem Wirbelpfeifen-Strömungsmesser (23) durch ein zweites Tangentialrohr (42) verbunden ist.

7. Vorrichtung nach einem der Ansprüche 1-6, wobei der Messwertwandler (7) ein Mikrofon oder ein piezoelektrischer Messwertwandler ist.

8. Vorrichtung nach einem der Ansprüche 1-7, wobei der Wirbelpfeifen-Strömungsmesser die folgenden Abmessungen hat:

- $D_{in}$ von 4 bis 7 mm,
- $D_{out}$ von 6 bis 9 mm,
- $D_c$ von 25 bis 35 mm;
- $L_{out}$ von 4 bis 15 mm;

wobei $D_{in}$ den Durchmesser des tangentialen Einlasses (21) repräsentiert, $D_{out}$ den Durchmesser des axialen Auslasses (22) repräsentiert, $D_c$ den Durchmesser der Kammer (23) repräsentiert und $L_{out}$ die Länge des axialen Auslasses (22) repräsentiert.

9. Vorrichtung nach einem der Ansprüche 1-8, wobei elektrischer Strom durch mindestens eine Batterie oder durch eine externe Energiequelle über einen Energiekanal (11) zugeführt wird.

10. Vorrichtung nach Anspruch 9, wobei der Energiekanal (11) sowohl ein Kommunikations- als auch ein Energiekanal oder nur ein Kommunikationskanal ist.

11. Vorrichtung nach Anspruch 10, wobei der Kommunikations- und Energiekanal (11) eine Universal Serial Bus-Verbindung ist.

12. Vorrichtung nach einem der Ansprüche 1-10, wobei der Kommunikationskanal (11) eine drahtlose Verbindung für die Kommunikation ist und die Energieversorgung eine Batterie ist.

13. Vorrichtung nach einem der Ansprüche 1-10, wobei der Kommunikationskanal (11) ein Audio-Klinkenverbinder ist und die Energieversorgung eine Batterie ist.

14. Vorrichtung nach einem der Ansprüche 1-10, wobei der Kommunikations- und Energiekanal (11) ein Audio-Klinkenverbinder ist.

15. Vorrichtung nach einem der Ansprüche 1-14, die des Weiteren ein intelligentes Gerät mit einem Display (13) umfasst, das mit dem Mikroprozessor (10) anstelle des Displays (12) verbunden ist.

16. Vorrichtung nach Anspruch 15, wobei das intelligente Gerät (13) aus der Gruppe bestehend aus einem Smartphone, einem Laptop und einem Desktop-Personalcomputer ausgewählt ist.

17. Vorrichtung nach einem der Ansprüche 1-16, das des Weiteren ein Fahrzeug-Interlocksystem (14) umfasst, das mit dem Mikroprozessor (10) verbunden ist.

18. Vorrichtung nach einem der Ansprüche 15-16 oder Anspruch 17, wobei die Verbindung mit dem intelligenten Gerät (13) oder dem Fahrzeug-Interlocksystem (14) ein Datenkommunikationskanal (11) ist.

19. Vorrichtung nach Anspruch 18, wobei der Datenkommunikationskanal (11) leitungsgebunden oder drahtlos ist.

20. Fahrzeug, das die Vorrichtung nach einem der Ansprüche 17-19 umfasst.

21. Verfahren zum Steuern des Betriebes des Fahrzeugs nach Anspruch 20 in Abhängigkeit von der Blutalkohol-Konzentration des Fahrzeuglenkers, das Folgendes umfasst:

   a. Ermitteln der Blutalkohol-Konzentration des Fahrzeuglenkers durch Probennahme der Atemluft des Fahrzeuglenkers in die Vorrichtung nach einem der Ansprüche 17-19;
   b. Vergleichen der Blutalkohol-Konzentration mit einer ermittelten Alkoholkonzentration, die in dem Interlocksystem (14) eingestellt ist;
   c. Betreiben des Interlocksystems (14), um zu verhindern, dass der Benutzer das Fahrzeug startet, wenn die Blutalkohol-Konzentration mindestens so hoch ist wie die ermittelte Alkoholkonzentration.

22. Verfahren zum Betreiben der Vorrichtung nach einem der Ansprüche 1-16, das Folgendes umfasst:

   a. Versetzen der Vorrichtung in den START-Zustand (51);
   b. Bereitstellen einer ausgeatmeten Atemluftprobe über eine bestimmte Zeitdauer, in der Probennehmerdaten, in Form einer detektierten Frequenz f, erfasst werden;
   c. Umwandeln der Frequenz f in eine Strömungsrate F durch die invertierende Gleichung f = k·F;
   d. Überleiten der Vorrichtung in einen Entscheidungszustand (53), in dem, wenn die Strömung F größer ist als eine zuvor festgelegte Schwelle F2, die Vorrichtung in den anschließenden Zustand (54) übergeht und anderenfalls in den Zustand (52) zurückkehrt;
   e. im Zustand (54) werden sowohl der Wirbelströmungsmesser-Probennehmer als auch der Sensor über eine bestimmte Zeitdauer abgetastet;
   f. Überleiten der Vorrichtung in den Zustand (55), in dem die erfassten Strömungsdaten integriert werden, um die Gesamtvolumendaten zu erhalten, gemäß der Gleichung (2)

$$V(nT) = \sum_{k=1}^{n} F(kT) \cdot T$$

wobei F(kT) der Strömungswert ist, der mit der Gleichung (1) anhand der Frequenz f erhalten wird, die in dem k-ten Probennahmefenster gemessen wurde, T die Länge des Probennahmefensters ist, V(nT) das Volumen ist, das die Summe des ersten bis n-ten Probennahmefensters darstellt, und das erste Probennahmefenster genommen wird, wenn der Zustand (53) erstmals verifiziert wird, wodurch der Strömungsbeginn ermittelt wird;
   g. Überleiten der Vorrichtung in den Entscheidungszustand (56), in dem, wenn das Volumen V größer ist als eine zuvor festgelegte Schwelle $V_1$, die Vorrichtung in den Zustand (58) übergeht, und anderenfalls in den Zustand (57) geht; wobei in diesem Entscheidungszustand, wenn die Strömung F größer ist als eine zuvor festgelegte Schwelle F1, die Vorrichtung in den Zustand (54) zurückkehrt, in dem sie fortfährt, die zwei Werte abzutasten und zu integrieren, und anderenfalls in den KEIN-ERFOLG-Zustand (62) übergeht;

h. im Zustand (58) wird der Sensorstrom I erfasst und integriert, um die Gesamtbeladung Q zu erhalten, gemäß der Gleichung (3)

$$Q(nT) = \sum_{k=1}^{n} I(kT) \cdot T$$

wobei I(kT) der Sensorstrom ist, der in dem k-ten Probennahmefenster gemessen und optional gemittelt wird, T die Länge des Probennahmefensters ist, Q(nT) die Beladung ist, die die Summe des ersten bis n-ten Probennahmefensters darstellt, und das erste Probennahmefenster genommen wird, wenn der Zustand (53) erstmals verifiziert wird, wodurch der Strömungsbeginn ermittelt wird;

i. Überleiten der Vorrichtung in den Zustand (59), in dem der BrAC gemäß Gleichung (4) berechnet wird:

$$BrAC = K \cdot \frac{Q}{V}$$

wobei K ein Kalibrierungsparameter ist, Q die Beladung ist, die mittels Gleichung (3) erhalten wird, und V das Volumen ist, das mittels Gleichung (2) erhalten wird, und die Vorrichtung dann in den Zustand (60) übergeht, in dem das BrAC-Ergebnis angezeigt wird, oder übertragen oder zur weiteren Verwendung gespeichert wird, und der Messprozess dann mit dem ENDE (61) abgeschlossen wird;

j. Anzeigen der BrAC;

k. optionales Umwandeln der Atemluft-Alkoholkonzentration (BrAC) in eine Blutalkohol-Konzentration (BAC) und Anzeigen der BAC.

**23.** Verfahren nach Anspruch 22, das des Weiteren Folgendes umfasst:

l. Berechnen und Anzeigen der Erholungszeit.

## Revendications

**1.** Appareil pour la détection de la concentration d'éthanol dans l'haleine exhalée d'un sujet, comprenant

a. un ensemble (15), dans lequel il est fourni un canal d'haleine principal (1) pour l'échantillonnage de l'haleine, ledit canal principal (1) est connecté en parallèle à un canal secondaire (2), de sorte qu'une partie du flux principal de l'haleine échantillonnée passe dans le canal secondaire (2) et revient dans le canal principal ;

b. un capteur d'éthanol (3) placé dans le canal secondaire, de sorte que ladite partie de l'haleine échantillonnée passe dans ledit capteur ; ledit capteur (3) comprenant facultativement un capteur de température ;

c. un débitmètre sifflet à vortex (5) placé à l'extrémité du canal principal (1) pour recueillir l'haleine échantillonnée ;

d. un transducteur (7) situé dans le débitmètre sifflet à vortex (5) pour obtenir un signal électrique ;

e. deux amplificateurs (8), (9) connectés à titre d'entrée au capteur (3) et au transducteur (7), respectivement et à titre de sortie à un microprocesseur avec une mémoire contenant un algorithme de logiciel (10) pour le calcul de la concentration en alcool éthylique ;

f. un afficheur (12) connecté au microprocesseur (10).

**2.** Appareil selon la revendication 1, dans lequel ledit appareil est portable ou fixe.

**3.** Appareil selon la revendication 1 ou 2, dans lequel ladite concentration en éthanol détectée est directement corrélée à la concentration en éthanol dans le sang chez ledit sujet.

**4.** Appareil selon la revendication 3, dans lequel la concentration en éthanol dans l'haleine ou ladite concentration en éthanol dans le sang chez ledit sujet est affichée.

**5.** Appareil selon l'une quelconque des revendications 1 à 4, dans lequel ledit capteur d'éthanol (3) est un capteur à pile à combustible.

**6.** Appareil selon la revendication 1, dans lequel dans ledit ensemble (15), en alternative au point a. il est fourni :

a'. un canal d'haleine principal (1) pour l'échantillonnage de l'haleine, ledit canal principal (1) est connecté en série audit débitmètre sifflet à vortex (23), à partir duquel un premier tuyau tangentiel (41) connecté audit débitmètre sifflet à vortex (23) et au capteur (43), puis reconnecté audit débitmètre sifflet à vortex (23) par un second tuyau tangentiel (42).

**7.** Appareil selon l'une quelconque des revendications 1 à 6, dans lequel ledit transducteur (7) est un microphone ou un transducteur piézoélectrique.

**8.** Appareil selon l'une quelconque des revendications 1 à 7, dans lequel ledit débitmètre sifflet à vortex a les dimensions suivantes :

$D_{in}$ de 4 à 7 mm,
$D_{out}$ de 6 à 9 mm,
$D_c$ de 25 à 35 mm ;
$L_{out}$ de 4 à 15 mm ;

dans lequel $D_{in}$ représente le diamètre de l'entrée tangentielle (21), $D_{out}$ représente le diamètre de la sortie axiale (22), $D_c$ représente le diamètre de la chambre (23) et $L_{out}$ représente la longueur de la sortie axiale (22).

**9.** Appareil selon l'une quelconque des revendications 1 à 8, dans lequel l'alimentation est fournie par au moins une batterie ou par une source d'alimentation externe à travers un canal d'alimentation (11).

**10.** Appareil selon la revendication 9, dans lequel ledit canal d'alimentation (11) est également un canal de communication et d'alimentation ou uniquement un canal de communication.

**11.** Appareil selon la revendication 10, dans lequel ledit canal de communication et d'alimentation (11) est une connexion par bus série universel.

**12.** Appareil selon l'une quelconque des revendications 1 à 10, dans lequel ledit canal de communication (11) est une connexion sans fil pour la communication et une batterie pour l'alimentation.

**13.** Appareil selon l'une quelconque des revendications 1 à 10, dans lequel ledit canal de communication (11) est un connecteur jack audio et une batterie pour l'alimentation.

**14.** Appareil selon l'une quelconque des revendications 1 à 10, dans lequel ledit canal de communication et d'alimentation (11) est un connecteur jack audio.

**15.** Appareil selon l'une quelconque des revendications 1 à 14, comprenant en outre un dispositif intelligent avec afficheur (13) connecté au microprocesseur (10) au lieu de l'afficheur (12).

**16.** Appareil selon la revendication 15, dans lequel ledit dispositif intelligent (13) est sélectionné dans le groupe constitué par un smartphone, un ordinateur portable et un ordinateur de bureau.

**17.** Appareil selon l'une quelconque des revendications 1 à 16, comprenant en outre un système de verrouillage de véhicule (14) connecté audit microprocesseur (10).

**18.** Appareil selon l'une quelconque des revendications 15 et 16 ou de la revendication 17, dans lequel la connexion audit dispositif intelligent (13) ou audit système de verrouillage de véhicule (14) est un canal de communication de données (11).

**19.** Appareil selon la revendication 18, dans lequel ledit canal de communication de données (11) est câblé ou sans fil.

**20.** Véhicule comprenant l'appareil selon l'une quelconque des revendications 17 à 19.

**21.** Procédé pour la commande de l'utilisation du véhicule selon la revendication 20 en fonction de la concentration en alcool dans le sang de l'utilisateur du véhicule, comprenant :

    a. la détermination de la concentration en alcool dans le sang dudit utilisateur du véhicule, en échantillonnant l'haleine dudit utilisateur dans l'appareil selon l'une quelconque des revendications 17 à 19 ;

    b. la comparaison de ladite concentration en alcool dans le sang à une concentration en alcool déterminée établie dans ledit système de verrouillage (14) ;

    c. l'utilisation dudit système de verrouillage (14), afin d'empêcher l'utilisateur de démarrer le véhicule, si ladite concentration en alcool dans le sang est égale ou supérieure à ladite concentration en alcool déterminée.

**22.** Procédé d'utilisation de l'appareil selon l'une quelconque des revendications 1 à 16, comprenant :

    a. le placement de l'appareil dans la condition DÉPART (51) ;

    b. la fourniture d'un échantillon d'haleine exhalée pendant une certaine durée, dans laquelle des données de l'échantillonneur, sous la forme de la fréquence détectée $f$ sont recueillies ;

    c. la conversion de la fréquence $f$ en débit $F$ en inversant l'équation $f = k \cdot F$ ;

    d. le passage dudit appareil à une condition décisionnelle (53) dans laquelle, si le flux F est supérieur à un seuil prédéfini F2, l'appareil passe à la condition suivante (54), dans le cas contraire il revient à la condition (52) ;

    e. dans la condition (54), tant l'échantillonneur du débitmètre à vortex et le capteur sont échantillonnés pendant une certaine durée ;

    f. le passage dudit appareil à la condition (55) dans laquelle les données de flux acquises sont intégrées pour obtenir les données de volume total selon l'équation (2)

$$V(nT) = \sum_{k=1}^{n} F(kT) \cdot T$$

dans laquelle $F(kT)$ est la valeur de flux récupérée avec l'équation (1) à partir de la fréquence $f$ mesurée dans la *kième* fenêtre d'échantillonnage, $T$ est la longueur de la fenêtre d'échantillonnage, $V(nT)$ est le volume additionné de la première à la nième fenêtre d'échantillonnage, et la première fenêtre d'échantillonnage est prise quand la condition (53) est la première vérifiée, et détermine donc le début du flux ;

    g. le passage dudit appareil à la condition décisionnelle (56) dans laquelle, si le volume $V$ est supérieur à un seuil prédéfini $V_1$, l'appareil passe à la condition (58), dans le cas contraire il passe à la condition (57) ; dans cette condition décisionnelle, si le flux $F$ est supérieur à un seuil prédéfini F1 l'appareil revient à la condition (54) dans laquelle il continue à échantillonner et à intégrer les deux valeurs, dans le cas contraire il passe à la condition ÉCHEC (62) ;

    h. dans la condition (58), l'acquisition du courant de capteur $I$ et son intégration pour obtenir la charge totale $Q$ selon l'équation (3)

$$Q(nT) = \sum_{k=1}^{n} I(kT) \cdot T$$

dans laquelle $I(kT)$ est le courant de capteur mesuré et facultativement moyenné dans la kième fenêtre d'échantillonnage, $T$ est la longueur de la fenêtre d'échantillonnage, $Q(nT)$ est la charge additionnée de la première à la nième fenêtre d'échantillonnage, et la première fenêtre d'échantillonnage est prise quand la condition (53) est la première vérifiée, et détermine donc le début du flux ;

    i. le passage dudit appareil à la condition (59) dans laquelle la BrAC est calculée en fonction de l'équation (4)

$$BrAC = K \cdot \frac{Q}{V}$$

dans laquelle K est un paramètre d'étalonnage, Q est la charge obtenue à partir de l'équation (3) et V est le volume obtenu à partir de l'équation (2), l'appareil passe alors à la condition (60) dans laquelle le résultat de BrAC est affiché, ou transmis ou stocké pour une utilisation ultérieure, le procédé de mesure conclut alors à la FIN (61) ;

    j. l'affichage de la BrAC ;

    k. la conversion facultative de la concentration en alcool dans l'haleine (BrAC) en concentration en alcool dans le sang (BAC) et l'affichage de ladite BAC.

**23.** Procédé selon la revendication 22, comprenant en outre :

l. le calcul et l'affichage du temps de récupération.

Fig. 1

Fig. 2

Fig. 3

Fig. 4

**Fig. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1046910 A
- EP 0437055 A
- EP 0834072 A
- US 6026674 A
- US 3966579 A
- US 20110009765 A
- WO 2012087186 A
- US 4314564 A
- WO 2012087187 A
- US 2013021153 A
- GB 2018596 A
- US 2794341 A

### Non-patent literature cited in the description

- *ACM DEV'10,* 17 December 2010
- Vortex flowmeter with enhanced accuracy and reliability by means of sensor fusion and self-validation. **MENZ B.** MEASUREMENT. INSTITUTE OF MEASUREMENT AND CONTROL, 12 November 1997, vol. 22, 123-128
- **SATO, H.** *IEEE S 0018-9456(00)02236-1*
- **SATO, H.** *SICE TR 0007/99/3507-0840*